# EUROPEAN PATENT APPLICATION

(11) **EP 1 331 277 A1**
(43) Date of publication of application: **30.07.2003**
(21) Application number: 03075143.2
(22) Date of filing: 17.01.2003
(51) Int. Cl.: C12Q 1/68

(54) **Method for DNA sequencing and gene identification**

(30) Priority: 29.01.2002 US 59988
(71) Applicant: EASTMAN KODAK COMPANY, Rochester, New York 14650 (US)
(72) Inventor: Yang, Zhihao, Rochester, New York 14650-2201 (US); Qiao, Tiecheng A., Rochester, New York 14650-2201 (US)
(74) Representative: Haile, Helen Cynthia

(57) **Abstract**

A method for single molecule identification of a target DNA molecule in a random coil state having the following steps: a) attaching an optically distinguishable material to a DNA sequence recognition unit; b) hybridizing the DNA sequence recognition unit to the target DNA molecule in a random coil state to form a hybridized DNA complex in a random coil state; c) stretching the hybridized DNA complex in a random coil state to form a hybridized DNA complex in a substantially linear configuration; and d) detecting the optically distinguishable material in a sequential manner along the substantially linear hybridized DNA complex, thereby identifying the target DNA molecule.

## Description

This invention relates to a method for identifying a target DNA molecule.

With the human genome project moving to the post genomic sequencing era, techniques such as single nucleotide polymorphism analysis, genomic function analysis, and proteome analysis have found wide spread applications. However, important technical challenges remain such as DNA sequencing or gene identification speed, length of the DNA that can be read during a single sequencing run, and the amount of nucleic acid template required. These factors suggest the preference of sequencing the genetic information of single cells without prior amplification and without prior need to clone the genetic materials into sequencing vectors. Practical methods in single molecule detection (SMD) for sequencing DNA or identifying characteristic genetic segments in a single chromosome, with high speed, highly-automated, and long read lengths are highly needed.

There are two traditional techniques for sequencing DNA: 1) the dideoxy termination method developed by Sanger et al. (*Proc. Natl. Acad. Sci. U.S.A.* 74, 5467 (1977)), and 2) the Maxam-Gilbert chemical degradation method developed by Maxam and Gilbert (*Proc. Natl. Acad. Sci. U.S.A.* 74, 564 (1977)). Both methods involve either ultrathin slab gel electrophoresis or capillary array electrophoresis techniques, which are labor-intensive and time-consuming, and require extensive pretreatment of the sample DNA. More recently, methods using dyes or fluorescent labels associated with the terminal nucleotide have been developed; however, the sequencing is still done with gel electrophoresis and automated fluorescent detectors.

Soper et al., in U.S. Patent No. 5,846,727, have disclosed a method that uses a single-mode optical fiber to direct the excitation light to the capillary channel, and the fluorescence signals are detected with a second single-mode optical fiber. The Soper et al. method requires polymerase chain reaction (PCR) amplification of a template DNA, and purification and gel electrophoresis of oligonucleotide sequencing ladders prior to initiation of the separation reaction. These procedures require significant quantities of a target DNA.

Several attempts towards single molecular DNA sequencing or detection have been made. For example, Goodwin et al. in "Application of Single Molecule Detection to DNA Sequencing" *Nucleos. Nucleot.* 16, 543, (1991), described a method of using DNA polymerase to synthesize a complete complementary strand which incorporates four different fluorescently labeled deoxyribonucleotide triphosphate (dNTP) analogs, and sequentially releases individual fluorescently labeled dNTPs using exonuclease. In this method, both polymerase and exonuclease have to show activity on a highly modified DNA strand, and a DNA strand substituted with four different fluorescent dNTP has to be generated.

In addition, the previous attempts in single molecular DNA sequencing, as disclosed in U.S. Patents 5,209,834, 4,962,037 and 5,405,747, all use fluorescent molecules as labels, and thus have to face the difficulties in single fluorescent molecule detection techniques, which are found to be quite complicated and challenging as described in U.S. Patent 6,049,380 of Goodwin et al.

Other approaches to the SMD of DNA include using scanning probe microscopy to determine the spatial sequence of fixed and stretched DNA molecules on a substrate as disclosed by Hansma et al. *(Science, 256,* 1180, (1992)). However, there is a problem with this method since the narrow spacing of bases in DNA molecules and the small physicochemical differences among the bases has to be differentiated. It is also difficult for such a method to become fast and with a high throughput.

It is an object of this invention to provide a method for single molecule identification of a target DNA molecule.

This and other objects are achieved in accordance with this invention which comprises a method for single molecule identification of a target DNA molecule in a random coil state comprising the following steps:
a) attaching an optically distinguishable material to a DNA sequence recognition unit;
b) hybridizing the DNA sequence recognition unit to the target DNA molecule in a random coil state to form a hybridized DNA complex in a random coil state;
c) stretching the hybridized DNA complex in a random coil state to form a hybridized DNA complex in a substantially linear configuration; and
d) detecting the optically distinguishable material in a sequential manner along the substantially linear hybridized DNA complex, thereby identifying the target DNA molecule.

The stretching step c) above can also be performed first in the process so that the process would be as follows:
a) stretching the target DNA molecule in a random coil state to form a substantially linear configuration; and
b) attaching an optically distinguishable material to a DNA sequence recognition unit;
c) hybridizing the DNA sequence recognition unit to the target DNA molecule in a substantially linear configuration to form a hybridized DNA complex in a substantially linear configuration;
d) detecting the optically distinguishable material in a sequential manner along the substantially linear hybridized DNA complex, thereby identifying the target DNA molecule.

By use of the invention, a SMD of a target DNA molecule can be identified in a fast and efficient manner.

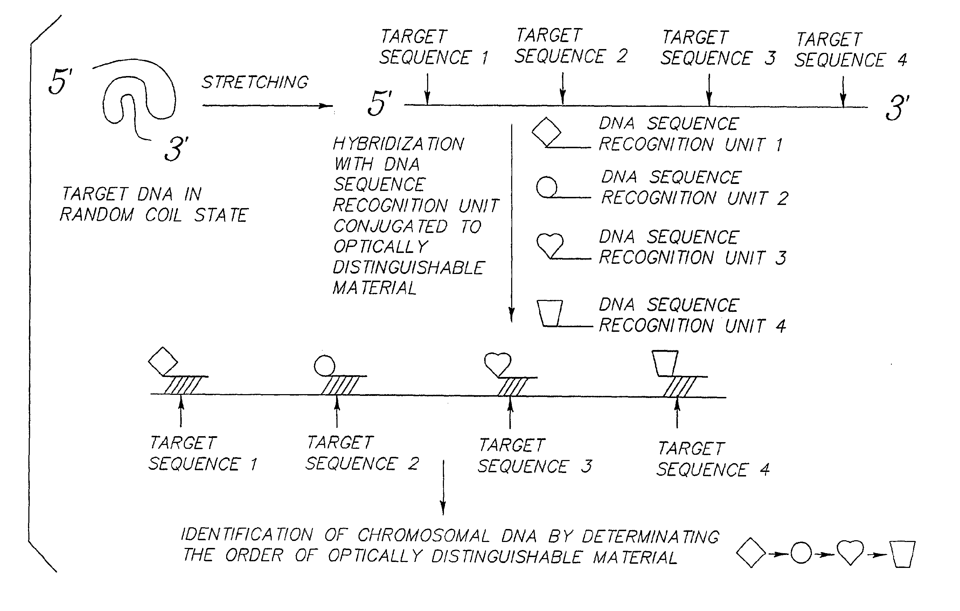

Fig. 1 is a schematic representation showing how a target DNA in a random coil state can be stretched and hybridized with a series of DNA recognition units conjugated with optically distinguishable materials.

The international collective effort on whole genome sequencing of various organisms has resulted in the deposition of hundreds of bacterial and viral genome sequences into a gene bank data base. The establishment of such a publicly accessible data base make it extremely easy to get access to the whole genome sequence of many disease bacteria and viruses through their accession numbers, e.g., gram-negative bacterium *Escherichia coli* O157:H7 strain EDL933, as described in the January 25, 2001 issue of Nature (accession number AE005177), and gram-positive bacterium *Bacillus subtilis,* as described in the November 20, 1997 issue of Nature (accession number AL009126). Once a bacterium or virus genome sequence is known, it is possible to design multiple gene or DNA sequence recognition units, which are specifically, targeted on the unique nucleic acid fragments of the bacterium or virus genome. Such a designed gene or DNA sequence recognition unit can be easily made using an automatic DNA synthesis machine and covalently attached to an optically distinguishable material. Therefore, there exists a library, which contains known DNA sequence recognition units.

A DNA molecule consists of four bases, A, T, G, and C, which are connected in linear manner covalently. The interaction among four bases follows the "Watson-Crick" base paring rule of A to T and G to C mediated by hydrogen bonds. When two single strand DNA molecules having a perfect "Watson-Crick" base paring match, they are referred as a complementary strand. The interaction between two complementary strands is termed hybridization. Sometimes complementary strands may contain one or more base-pairing mismatches as well.

The present invention provides a novel approach to the SMD of a DNA molecule utilizing a known library of DNA sequence recognition units attached to a variety of optically distinguishable materials. When such optically distinguishable material attached DNA sequence recognition units are allowed to hybridize to a target DNA molecule intended to be identified, a series of optically distinguishable materials will associate with a target DNA molecule at a specific sequence location through hybridization between DNA sequence recognition units and their complementary sequence fragment on the target DNA molecule. When the hybridized target DNA molecule is stretched from a random coil to a linear state, then the optically distinguishable material can be determined in a linear sequential manner. Therefore the genetic sequence information and the identity of the target DNA molecule can be obtained.

Some commonly used DNA sequence recognition units which can used in the invention include, for example, DNA and DNA fragments, synthetic oligonucleotides, and peptide nucleic acids. In another embodiment of the invention, the DNA sequence recognition units can be any protein scaffold or synthetic molecular moiety capable of recognizing a specific DNA sequence.

The invention can be used to rapidly identify bacteria or viruses and genes.

Optically distinguishable materials which can be used in the invention include, for example, colored microparticles, such as, dyes, dye aggregates, pigments or nanocrystals; or microparticles, such as polymers or inorganic materials, having different shapes, such as curvilinear, spherical, donut shaped, elliptical, cubic, rod, etc. In a preferred embodiment of the invention, the optically distinguishable material comprises polymeric microparticles colored with a dye.

A method for coloring a microparticle has been described by L.B. Bangs in *"Uniform Latex Particles;*" Seragen Diagnostics Inc. 1984. Another approach to coloring a microparticle with dye is by covalently coupling one or more dyes to the surface of the microparticles. Examples for this approach can be found in U.S Patents 5,194,300 and 4,774,189. Colorants and pigments can also be incorporated into microparticles using micro-encapsulation methods as described in U.S. Patents 5,073,498 and 4,717,655. These methods can be performed by anyone skilled in the art.

Suitable methods for preparing polymeric particles are emulsion polymerization, as described in "Emulsion Polymerization" by I. Piirma, Academic Press, New York (1982) or by limited coalescence as described by T. H. Whitesides and D. S. Ross in *J. Colloid Interface Science,* vol. 169, pages 48-59, (1985). The particular polymer employed to make the particles or microparticles is usually a water immiscible synthetic polymer that may be colored, such as any amorphous water immiscible polymer. Examples of polymers that are useful include polystyrene, poly(methyl methacrylate) and poly(butyl acrylate). Copolymers such as a copolymer of styrene and butyl acrylate may also be used. In a preferred embodiment of the invention, the microparticles have a particle size of from 0.001 µm to 10 µm, preferably from 0.05 µm to 1 µm.

In another preferred embodiment of the invention, the DNA sequence recognition units are chemically attached to the optically distinguishable materials. The attachment of DNA sequence recognition units to the surface of microparticles can be performed according to the published procedures in the art (Bangs Laboratories, Inc, Technote #205). Some commonly used attachment groups on the surface of the microparticles include carboxyl, amino, hydroxyl, hydrazide, amide, chloromethyl, epoxy, aldehyde, etc.

Other methods of attaching the optically distinguishable materials with DNA sequence recognition units include the use of bioactive links such as Biotin-Strepavidin bonding or antigen-antibody bonding.

In another preferred embodiment of the invention, more than one pair of optically distinguishable materials and their conjugated DNA sequence recognition units are used in determining or identifying the characteristic genomic information of a DNA molecule.

Large DNA molecules, like all macromolecules, have a random coil configuration under a non-perturbed condition. Many methods are known for stretching DNA molecules from a random coil configuration to a linear state. For example, DNA molecules may be stretched using a mechanical means such as applying a microscopic hydrodynamic force generated by microfluidic flows. These flows can be generated in simple microfluidic devices either via electrophoretic, electro-osmotic, or pressure-driven. When a large DNA molecule in solution passes with an elongational flow associated with acceleration of the fluid from a reservoir into a microfluidic channel, the DNA molecule can be oriented and stretched into linear state for at least a fraction of a second, as more fully described in copending U.S. Patent Application Serial Number 10/086,087 (Docket 83426).

Other methods for stretching a DNA molecule to a linear or substantially linear state include the use of optical tweezers (S. Chu, *Science,* 253, 861 (1991)), magnetic tweezers (B. Maier, et al. *Proc. Natl. Acad. Sci. U. S. A.,* 97,12001 (2000)) and atomic force microscope (M. Rief, et al. *Science,* 276, 1109 (1997), H. Li, et. al. *Proc. Natl. Acad. Sci. U. S. A.,* 10682 (2001)).

Fig. 1 schematically shows how to use a mixture of such optically distinguishable materials conjugated with DNA sequence recognition units to identify bacterial or viral chromosomal DNA. First of all, a chromosomal DNA from a bacterium or virus was isolated and stretched from random coil state to a linear state. This can be done by using one of the DNA stretching methods as described above. Secondly, a mixture of optically distinguishable materials conjugated with DNA sequence recognition units with sequences complementary to some gene fragment sequences of the target DNA intended to be identified was allowed to hybridize with linear stretched DNA. Thirdly, upon the completion of the hybridization event, the order of optically distinguishable materials hybridized to the linearly stretched target DNA was determined. Since each bacterium or virus has its unique chromosomal DNA sequence, the order determination of the optically distinguishable markers should unambiguously detect a bacterium or virus intended to be identified.

The following example is provided to illustrate the invention.

This example illustrates the attachment of a pre-synthesized single strand oligonucleotide as a DNA sequence recognition unit to the surface of a rnicroparticle, and the detection of a fluorescence signal due to the hybridization between a DNA recognition unit on the surface of such modified microparticles and its fluorescently labeled complementary single strand target DNA, in order to demonstrate the feasibility of the invention.

One hundred microliters of microparticle (4% w/v) was rinsed three times in an acetate buffer (0.01 M, pH5.0), and combined with one hundred microliters of 20 mM 2-(4-Dimethylcarbomoyl-pyridino)-ethane-1-sulfonate and ten percent of polyethyleneimine. The mixture was agitated at room temperature for one hour and rinsed three times with sodium boric buffer (0.05 M, pH8.3). The beads were re-suspended in a sodium boric buffer.

A 22-mer oligonucleotide DNA sequence recognition unit with 5'-amino-C6 modification was dissolved in one hundred microliters of sodium boric buffer to a final concentration of 40 nmol. 20 microliters of cyanuric chloride in acetonitrile was added to the DNA sequence recognition unit solution and the total volume was brought up to 250 microlites using a sodium boric buffer. The solution was agitated at room temperature for one hour and then dialyzed against one liter of boric buffer at room temperature for three hours.

100 microliters of the dialyzed DNA solution was mixed with 200 microliters of the bead suspension. The mixture was agitated at room temperature for one hour and rinsed three times with a sodium phosphate buffer (0.01 M, pH7.0).

A 22-mer oligonucleotide DNA with a 5'-fluorescein label, which has a complementary sequence to the 22-mer DNA sequence recognition unit, was dissolved in a hybridization solution (6XSSPE-SDS) containing 0.9 M NaCI, 0.06 M NaH₂PO₄, 0.006 M ethylenediamine tetraacetic acid, and 0.1% SDS, pH 7.6 to a final concentration of 1M. The 22-mer oligonucleotide DNA sequence recognition unit attached to the microparticle was hybridized in the hybridization solution starting at 68°C and slowly cooled down to room temperature. Following hybridization, the microparticles were washed in 0.5XSSPE-SDS for 15 minutes three times. The fluorescence image of the microparticles was obtained using an Olympus BH-2 microscope (Diagnostic Instruments, Inc. SPOT camera, CCD resolution of 1315 x 1033 pixels) with DPlanapo40 UV objective, mercury light source, blue excitation & barrier filters.

The above example demonstrates the feasibility of coupling a DNA recognition unit, a 22-mer synthetic oligonucleotide, to an optically distinguishable material-microparticle, and the capability of detecting the hybridization event between the DNA recognition unit and a sequence complementary target DNA molecule, a 22-mer oligonucleotide DNA with 5'-fluorescein label.

Furthermore, a dye can be incorporated into the microparticles as described above to produce population and sub-population of optically distinguishable materials, which subsequently can be coupled to different DNA recognition units. Since it has been demonstrated that such a DNA recognition unit associated with an optically distinguishable material can hybridize to a target DNA molecule with a complementary sequence, using one of the methods to stretch a DNA molecule, the hybridization complex can be stretched into a linear configuration to allow the detection of a series of optically distinguishable materials in a sequential manner along the linear hybridized DNA complex, thereby identifying the target DNA molecule.

Alternatively, a target DNA molecule can also be stretched first, and then hybridized with a series of corresponding DNA recognition units coupled to the optically distinguishable materials. Variations of actual operation procedure can be modified by one skilled in the art.

## Claims

1. A method for single molecule identification of a target DNA molecule in a random coil state comprising the following steps:
a) attaching an optically distinguishable material to a DNA sequence recognition unit;
b) hybridizing said DNA sequence recognition unit to said target DNA molecule in a random coil state to form a hybridized DNA complex in a random coil state;
c) stretching said hybridized DNA complex in a random coil state to form a hybridized DNA complex in a substantially linear configuration; and
d) detecting said optically distinguishable material in a sequential manner along said substantially linear hybridized DNA complex, thereby identifying said target DNA molecule.

2. The method of claim 1 wherein said optically distinguishable material comprises colored microparticles.

3. The method of claim 1 wherein said optically distinguishable material comprises microparticles having different shapes.

4. The method of claim 2 wherein said colored microparticles comprise dyes, dye aggregates, pigments or nanocrystals.

5. The method of claim 1 wherein said DNA sequence recognition unit comprises DNA, DNA fragments, synthetic oligonucleotides or peptide nucleic acids.

6. The method of claim 1 wherein said DNA sequence recognition units comprise any protein scaffold or synthetic molecular moiety capable of recognizing a specific DNA sequence.

7. The method of claim 1 wherein said stretching of said hybridized DNA complex in a random coil state to form a hybridized DNA complex in a substantially linear configuration is accomplished by using a mechanical means.

8. A method for single molecule identification of a target DNA molecule in a random coil state comprising the following steps:
a) stretching said target DNA molecule in a random coil state to form a substantially linear configuration;
b) attaching an optically distinguishable material to a DNA sequence recognition unit;
c) hybridizing said DNA sequence recognition unit to said target DNA molecule in a substantially linear configuration to form a hybridized DNA complex in a substantially linear configuration; and
d) detecting said optically distinguishable material in a sequential manner along said substantially linear hybridized DNA complex, thereby identifying said target DNA molecule.

9. The method of claim 8 wherein said optically distinguishable material comprises colored microparticles.

10. The method of claim 8 wherein said optically distinguishable material comprises microparticles having different shapes.
